# EUROPEAN PATENT SPECIFICATION

(11) **EP 1 682 228 B1**
(45) Date of publication and mention of the grant of the patent: **02.12.2009**
(21) Application number: 04818753.8
(22) Date of filing: 28.10.2004
(51) Int. Cl.: A61K 8/893, A61Q 17/04

(54) **LIGHT PROTECTING COMPOSITION WITH REDUCED TOTAL AMOUNT OF UV FILTER CONTAINING A POLYSILOXANE-BASED UV FILTER**
LICHTSCHUTZZUSAMMENSETZUNG MIT VERRINGERTER GESAMTMENGE AN UV-FILTER MIT EINEM UV-FILTER AUF POLYSILOXAN-BASIS
COMPOSITION DE PROTECTION CONTRE LA LUMIERE AYANT UNE TENEUR TOTALE LIMITEE EN FILTRE UV ET CONTENANT UN FILTRE UV A BASE DE POLYSILOXANE

(30) Priority: 05.11.2003 EP 03025408
(43) Date of publication of application: 26.07.2006
(73) Proprietor: DSM IP Assets B.V., 6411 TE Heerlen (NL)
(72) Inventor: RUDOLPH, Thomas, 64291 Darmstadt (DE); TRAEGER, Christina, 40597 Düsseldorf (DE); BERG, Katja, CH-4303 Kaiseraugst (CH); SCHEHLMANN, Volker, 79650 Schopfheim (DE); WESTENFELDER, Horst, 67435 Neustadt a.d.W. (DE)
(74) Representative: Best, Michael
(86) International application number: PCT/EP2004/012194
(87) International publication number: WO 2005/048960

(56) References cited:
- EP-A- 0 897 715
- EP-A- 0 979 645
- US-A- 5 403 944
- US-B1- 6 193 959

## Description

There is a constantly increasing need for sunscreen protecting agents in a population that is exposed to an increasing amount of damaging sunlight. Repetitive sun exposure can result in skin changes known as photoaged skin. The clinical changes that are seen in photoaged skin differ from those of normally aged skin in sunlight protected sites of the body. Among the damaging results of extensive sun exposure of the skin, there is increased wrinkling, elastosis, pigmentary changes, precancerous and cancerous skin lesions, etc.

Accordingly, many sunscreening compounds have been developed in the past protecting against the harmful effect of UV-A (320-400 nm) and/or UV-B (290-320 nm) wavelength and even shorter wavelength (UV-C filters).

Particularly successful UV filters are recently developed UV filters which are based on chromophore-bearing polysiloxanes which may be either linear or cyclic and which are described e.g. in WO 93/04665, WO 94/06404, EP-B 538 431, EP-A 392 883 and in EP 358 584.

In EP 897715 it has been shown that sunscreens containing combinations of (a) UV-absorbing organic compounds which are solid under normal conditions with (b) chromophore-bearing polysiloxanes or cyclosiloxanes (based on periodically recurring SiO-elements) show higher light protection factors than sunscreens containing the organic compounds alone. As specific combinations of UV-absorbing organic compounds which are solid under normal conditions with chromophore-bearing polysiloxanes or cyclosiloxanes (based on periodically recurring SiO-elements) EP 897 715 exemplifies only combinations containing a "Polymerfilter X" and a triazine compound or 2-phenylbenzimidazole-5-sulfonic acid or their salts. These organic UV-filters do not have bulky substituents. The UV-filter combinations disclosed in EP 897 715 have a good light protecting factor, but the triazine compounds are not very soluble, and there is still a need for UV-filter combinations having a comparable or preferably higher light protecting factor than the UV-filter combinations disclosed in EP 897 715. All examples of EP 897 715 which employ a UV-absorbing organic compound having bulky (sterically demanding) substituents also comprise a triazine compound which does not have bulky (sterically demanding) substituents. This indicates to a skilled person that the presence of a UV-absorbing compound having bulky or sterically demanding substituents alone is considered as not being sufficient for increasing the, lightprotecting factor of chromophore-bearing polysiloxanes or cyclosiloxanes (based on periodically recurring SiO-elements), but that in these compositions an additional triazine compound must be present.

In the prior art several combinations of chromophore-bearing polysiloxanes or cyclosiloxanes with other UV-absorbing organic compounds are disclosed, but all the UV-absorbing organic compounds which were considered useful for combining with the polysiloxanes or cyclosiloxanes are UV-absorbing organic compounds which did not have bulky or sterically demanding substituents. As typical examples of such documents it can be referred to EP 848 945 which discloses combinations of a polysiloxane, a triazine and a dibenzoylmethane derivative (DBM), to EP-A 709 980 which discloses photostable compositions of polysiloxanes and DBM and to EP 979 645 which discloses a synergistic effect between linear or cyclic polysiloxanes and 2-phenylbenzimidazole-sulphonic acid or salts thereof (PBSA).

Thus, on the basis of the prior art documents it must be assumed that only UV-absorbing organic compounds which do not have bulky or sterically demanding substituents are suitable for mixing with chromophore-bearing polysiloxanes or cyclosiloxanes.

While there is a high demand for lightprotecting compositions having a high protection performance, there is on the other hand also dermatological as well as economic demands for reducing the total UV filter amount while the protecting performance of the composition can be maintained or even increased. Protecting performance of the composition as described here covers several aspects such as the sunprotecting factor, water resistance, UVA protection, UVA index or UVA balance [EP1291640, DIN87502]. It would be in particular advantageous to have a method for reducing the total UV filter amount while the protection performance of the composition can be maintained.

The present invention is based on the unexpected finding that a sunscreen composition containing a polysiloxane having a chromophore residue of the benzalmalonate type and at least one additional UV filter where the chromophore contains appropriate bulky (sterically demanding) substituents according to claim 1 provides synergistically enhanced protection indices. Therefore, the present application provides an advantageous method for significantly increasing the ratio of the *sunprotecting factor* to the *total amount of UV filters* of a lightprotecting composition.

In a preferred embodiment the light protecting composition contains in addition to the polysiloxane and at least one UV filter which contains bulky substituents other UV filters which are liquid or not liquid or belong to the group of pigments such as microparticulated ZnO and/ or TiO₂, and the like.

In a more preferred embodiment the amount of additional UV filters which are liquid at room temperature (25°C) is reduced to a miniumum in the light protection composition or the light protecting composition contains only additional UV filter which are not liquid or belong to the group of pigments such as microparticulated ZnO and/ or TiO₂, and the like in order to further increase the ratio of the *sunprotecting factor* to the *total amount of UV filters.* The term "not liquid" in the sense of this application means UV filters which are either solid or a melt that can crystallize at room temperature (25°C).

Appropriate bulky (sterically demanding) substituents as steric protection groups include preferably substituents such as t-butyl, 1,1,3,3-dimethylbutyl, camphor, diethylamino or silyl residues e.g. 2-methyl-3-[1,3,3,3-tetramethyl-1-[(trimethylsilyl)oxy]disiloxanyl]propyl- or 4-tris(trimethylsilyloxysilylpropyloxy). More preferred are substituent groups such as t-butyl, 1,1,3,3-dimethylbutyl, camphor or silyl residues.

Accordingly, the present application provides a method to increase the ratio of the *sunprotecting factor* to the *total UV filter amount* of a light protecting composition containing
a) at least one polysiloxane-based UV filter,
b) at least one additional UV filter which chromophore contains appropriate bulky (sterically demanding) substituents,
c) a carrier for the components a), b) and optionally d), and optionally
d) additional UV filter(s)
   with the proviso that 4,4'4"-(1,3,5-triazine-2,4,6-triyltriimino)-tris-benzoic-acid-tris(2-ethylhexylester) is not present.

The additional UV filter(s) can be liquid or not liquid in a preferred embodiment the additional UV filter(s) are not liquid.

The present invention also provides a method to increase the ratio of the *sunprotecting, factor* to the *total UV filter amount* of a light protecting composition the method comprising
a) the addition of a polysiloxane-based UV filter in order to reduce the amount of a UV filter which is liquid at room temperature (25°C) by which the total UV filter amount will be reduced, and
b) the addition of a UV filter(s) containing bulky (sterically demanding) groups and, and optionally
c) the addition of UV filter(s) which are not liquid at room temperature (25°C)

Preferably, the compositions of the present invention do not contain triazine compounds, in particular they do not contain a compound selected from:
- 4,4'4"-(1,3,5-triazine-2,4,6-triyltriimino)-tris-benzoic acid-tris(2,ethylhexylester)
- 2,4-bis-{[4-(2-Ethyl-hexyloxy)-2-hydroxy]-phenyl}-6-(4-methoxyphenyl)-1,3,5-triazine,
- 2,4-bis-{[4-(3-sulfonato)-2-hydroxy-propyloxy)-2-hydroxy]-phenyl}-6-(4-methoxyphenyl)-1,3,5-triazine sodium salt,
- 2,4-bis-{[4-(3-(2-propyloxy)-2-hydroxy-propyloxy)-2-hydroxy]-phenyl}-6-(4-methoxyphenyl)-1,3,5-triazine,
- 2,4-bis-{[4-(2-ethyl-hexyloxy)-2-hydroxy]-phenyl}-6-[4-(2-methoxyethyl-carboxyl)-phenylamino]-1,3,5-triazine,
- 2,4-bis-{[4-(3-(2-propyloxy)-2-hydroxy-propyloxy)-2-hydroxy]-phenyl}-6-[4-(2-ethylcarboxyl)-phenylamino]-1,3,5-triazine,
- 2,4-bis-{[4-(2-ethyl-hexyloxy)-2-hydroxy]-phenyl}-6-(1-methyl-pyrrol-2-yl)-1,3,5-triazine,
- 2,4-bis-{[4-tris(trimethylsiloxy-silylpropyloxy)-2-hydroxy]-phenyl}-6-(4-methoxyphenyl)-1,3,5-triazine,
- 2,4-bis-{[4-(2"-methylpropenyloxy)-2-hydroxy]-phenyl}-6-(4-methoxyphenyl)-1,3,5-triazine and
- 2,4-bis-{[4-(1',1',1',3',5',5',5'-heptamethylsiloxy-2"-methyl-propoloxy)-2-hydroxy]-phenyl}-6-(4-methoxyphenyl)-1,3,5,-triazine.

The novel light protecting compositions contain as component a) a polysiloxane-based UV filter. Such compounds are known, and it can be referred e.g. to EP-A 538 431, EP-A 358 584, EP-A 392 883, WO 94/06404 and WO 93/04665 which disclose polysiloxane-based UV filters which can preferably be used in the lightprotecting compositions of the present invention. Regarding the definition of these polysiloxane-based UV filters and the methods for preparing these filters, it is referred to the above referenced publications, the content of which is included herein by reference.

The preferred polysiloxane-based UV filters are linear or cyclic polysiloxane compounds according to formula la or lb: 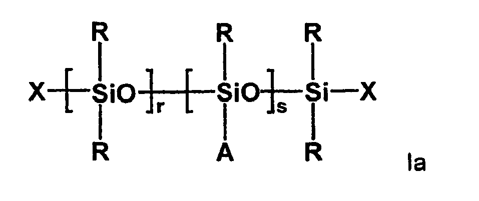 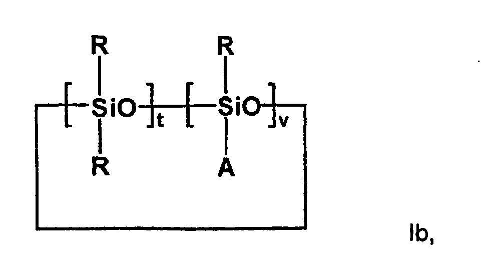 wherein
- X: is R or A;
- A: is selected from formula IIa and/ or IIb or IIc:

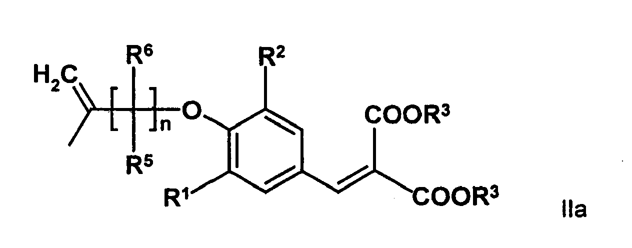

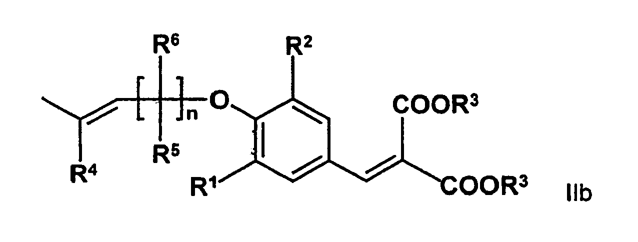

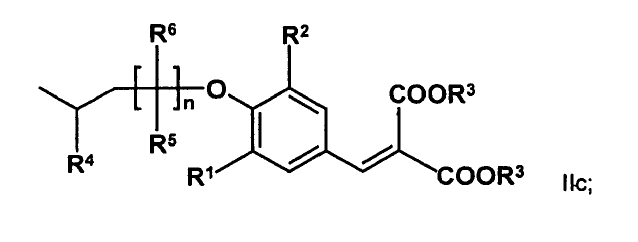

- R: is hydrogen, C₁₋₆-alkyl or phenyl;
- R¹ and R²: are each independently hydrogen, hydroxy, C₁₋₆-alkyl or -C₁₋₆-alkoxy;
- R³: is C₁₋₆-alkyl;
- R⁴: is hydrogen or C₁₋₆-alkyl;
- R⁵ and R⁶: are each independently hydrogen or C₁₋₆-alkyl;
- r: is from 0 to 250;
- s: is from 0 to 20;
- r + s: is at least 3;
- t: is from 0 to 10;
- v: is from 0 to 10;
- v + t: is at least 3; and
- n: is from 1 to 6;
with the proviso that when s is 0, at least one X is A.

The term "C₁₋₆-alkyl" refers to groups such as methyl, ethyl, propyl, isopropyl, butyl, sec. butyl, isobutyl, pentyl and neopentyl. The term "C₁₋₆-alkoxy" refers to the corresponding alkoxy groups.

The residues R are preferably methyl.

The residues R¹ and R² are preferably hydrogen, methoxy or ethoxy, more preferably hydrogen, or one of R¹ and R² is hydrogen and the other is methyl, methoxy or ethoxy.

The residues R³ are preferably methyl or ethyl, more preferably ethyl.

Preferably, R⁴ is hydrogen or methyl, R⁵ and R⁶ are hydrogen and n is 1.

The polysiloxane compounds having a group A of the general formula IIa and IIb and their preparation are described in European patent application EP-A 0 538 431. These polysiloxane compounds are most preferred.

The polysiloxane compounds having a group A of the general formula IIc and their preparation are described in the European patent application EP-A 0 358 584.

In the linear polysiloxane compounds according to formula la the chromophore carrying residue A may be connected to the end groups of the polysiloxane (X=A) or may be statistically distributed.

Linear polysiloxane compounds wherein the chromophore carrying residue A is statistically distributed are preferred. Said preferred polysiloxane compounds have at least one unit carrying the chromophore residue (s = 1), preferably s has a value of from about 2 to about 10, more preferably a statistical mean of about 4. The number of r of the other silicone units present in the polysiloxane compounds is preferably about 5 to about 150, more-preferably a statistical mean of about 60.

Polysiloxane compounds wherein 20% or less, preferably less than 10%, of the total siloxane units are units carrying a chromophore residue are preferred with respect to cosmetic properties.

The ratio of polysiloxane units having a chromophore residue A of the formula IIa to those having a chromophore residue A of the formula IIb is not critical. Said ratio may be about 1:1 to about 19:1, preferably about 2:1 to about 9:1, more preferably about 4:1.

The concentration of the polysiloxane compound in the cosmetic light screening composition is preferably about 2 to 20 wt%, more preferably about 5 wt%.

The polysiloxane compounds Ia or Ib wherein A is a reside of the formula IIa or IIb can be prepared as described in EP-B 0 538 431 by silylation of the corresponding benzalmalonates according to the following reaction scheme: 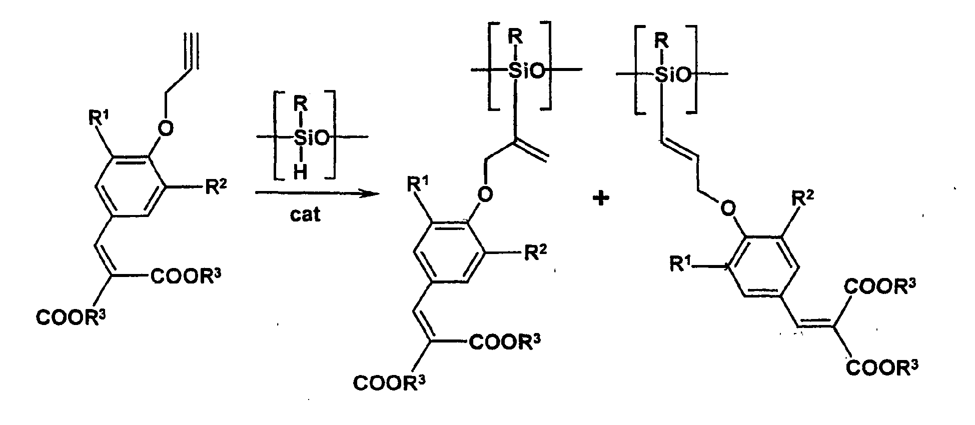 wherein R¹, R² and R³ are as defined above.

The silylation of the 4-(2-propinyloxy)phenyl methylene diethylester may be carried out employing known procedures for the addition of silicon bonded hydrogen atoms to groups containing aliphatic unsaturation. Such reactions are generally catalyzed by a platinum group metal or a complex of such a metal. Examples of catalysts which may be employed are platinum on carbon, chloroplatinic acid, platinum acetyl acetonate, complexes of platinum compounds with unsaturated compounds e.g. olefins and divinyl disiloxanes, complexes of rhodium and palladium compounds and complexes of platinum compounds supported on inorganic substrates. The addition reaction may be performed at reduced, atmospheric or increased pressure. A solvent can be used, e.g. toluene or xylene, in the reaction mixture although the presence of the solvent is not essential. It is also preferred to carry out the reaction at elevated reaction temperatures e.g. from about 50°C to about 150°C.

Particularly preferred are compounds of the general formula la, wherein
- X: signifies methyl,
- A: signifies the group of the formula IIa or IIb,

- R: signifies methyl,
- R¹ and R²: signify hydrogen, methoxy or ethoxy or one or R¹ and R² is hydrogen and the other is methyl, methoxy or ethoxy,
- R³: signifies methyl or ethyl,
- R⁴: signifies hydrogen or methyl,
- R⁵ and R⁶: signify hydrogen,
- r: is about 5 to 150,
- s: is about 2 to about 10, and
- n: has a value of 1.

Most preferred are linear polysiloxanes of the general formula la, wherein
- X: signifies methyl,
- A: signifies a group of the formula IIa or IIb,
- R: signifies methyl,
- R¹ and R²: signify hydrogen,
- R³: signifies ethyl,
- R⁴: signifies hydrogen,
- R⁵ and R⁶: signify hydrogen,
- r: is a statistical mean of about 60,
- s: is a statistical mean of about 4, and
- n: has a value of 1.

These most preferred linear polysiloxanes are commercially available under the tradename PARSOL SLX.

Examples of molecules containing bulky substituents are e.g. Butyl methoxydibenzoylmethane (Parsol 1789) (I), 4-Methylbenzylidenecamphor (PARSOL 5000) (II), 3-Benzylidenecamphor (Unisol S-22), Benzylidenecamphor sulfonic acid (Mexoryl SL), Homosalate (Neo HELIOPAN HMS) (V), Methylene bis-benzotriazo tetramethylbutylphenol (Tinosorb M) (III), Drometrizole Trisiloxane (Mexoryl XL) (IV), Champhor benzalkonium methosulfate (Mexoryl SK), and 2-(4-Diethylamino-2-hydroxy-benzoyl)-benzoic acid hexylester (VI) as described in the European Patent Publication EP 1046391 and the like:

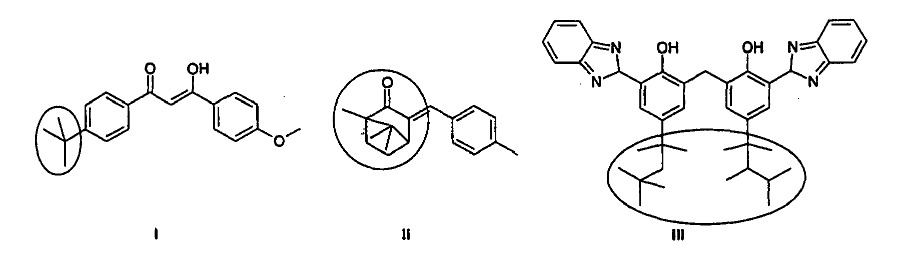

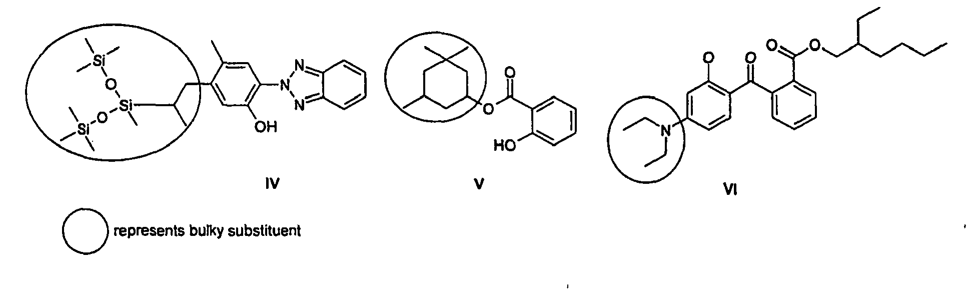

Additional information about these UV filters can be found e.g. in standard textbooks.

Particularly preferred are compounds I, II, III, IV and V above. Preferably, the bulky substituent contains a cyclohexane ring structure as in compounds II or V, Si-groups as in compound IV or carbon atoms with no hydrogen substituent as in compounds I and III.

Bulky groups as referred to in this inventions are substituents which provide sterical hindrance to a molecule such bulky (sterically demanding) substituents as steric protection groups include groups such as diethylamino, t-butyl, 1,1,3,3-dimethylbutyl, camphor, or silyl residues such as e.g. as 2-methyl-3-[1,3,3,3-tetramethyl-1-[(trimethylsilyl)oxy]disiloxanyl]propyl- or 4-tris(trimethylsilyloxysilylpropyloxy).

The concentration of the UV filter where the chromophore contains appropriate bulky substituents in the composition of the present invention is preferably 0.1 to 20 wt.%, more preferably 0.5 to 10 wt.%, such as 0.5 to 5 wt.%, e.g. about 1 or about 2 wt.%. The ratio of the siloxane-based UV filter and the UV filter where the chromophore contains appropriate bulky substituents depends on the effect on the sunprotecting factor which is to be achieved. For example, the ratio is about 20:1 to about 1:20, preferably 10:1 to 1:10, more preferably 5:1 to 1:5 such as about 1:1. Unsymmetrical mixtures can also be used.

The concentration of the additional UV filter in the composition of the present invention is preferably 0.1 to 20 wt.%, more preferably 0.5 to 10 wt.%, such as 0.5 to 5 wt.%, e.g. about 1 or about 2 wt.%. The ratio of the siloxane-based UV filter and the additional UV filter d) depends on the effect on the sunprotecting factor which is to be achieved. For example, the ratio is about 20:1 to about 1:20, preferably 10:1 to 1:10, more preferably 5:1 to 1:5 such as about 1:1. Unsymmetrical mixtures can also be used.

These additional screening agents are advantageously selected from among the compounds listed below without being limited thereto:

Examples of UV B or broad spectrum screening agents, i.e. substances having absorption maxima between about 290 and 340 nm, which come into consideration for combination with the compounds of the present invention are for example the following organic and inorganic compounds:
Acrylates such as 2-ethylhexyl 2-cyano-3,3-diphenylacrylate (octocrylene, PARSOL® 340), ethyl 2-cyano-3,3-diphenylacrylate and the like;
--- Cinnamate derivatives such as octyl methoxycinnamate (PARASOL® MCX), ethoxyethyl methoxycinnamate, diethanolamine methoxycinnamate (PARSOL® Hydro), isoamyl methoxycinnamate and the like as well as cinnamic acid derivatives bond to siloxanes;
**---** p-Aminobenzoic acid derivatives, such as p-aminobenzoic acid, 2-ethylhexyl p-dimethylaminobenzoate, N-oxypropylenated ethyl p-aminobenzoate, glyceryl p-aminobenzoate,
--- Benzophenones such as benzophenone-3, benzophenone-4, 2,2', 4, 4'-tetrahydroxy-benzophenone, 2,2'-dihydroxy-4,4'-dimethoxybenzophenone and the like;
--- Esters of Benzalmalonic acid such as di-(2-ethylhexyl) 4-methoxybenzalmalonate
--- Esters of 2-(4-ethoxy-anilinomethylene)propandioic acid such as 2-(4-ethoxy anilinomethylene)propandioic acid diethyl ester as described in the European Patent Publication EP 0895 776
--- Pigments such as microparticulated TiO₂, and the like. The term "microparticulated" refers to a particle size from about 5 nm to about 200 nm, particularly from about 15 nm to about 100 nm. The TiO₂ particles may also be coated by metal oxides such as e.g. aluminum or zirconium oxides or by organic coatings such as e.g. polyols, methicone, aluminum stearate, alkyl silane. Such coatings are well known in the art.
--- Imidazole derivatives such as e.g. 2-phenyl benzimidazole sulfonic acid and its salts (PARSOL®HS). Salts of 2-phenyl benzimidazole sulfonic acid are e.g. alkali salts such as sodium- or potassium salts, ammonium salts, morpholine salts, salts of primary, sec. and tert. amines like monoethanolamine salts, diethanolamine salts and the like.
--- Salicylate derivatives such as isopropylbenzyl salicylate, benzyl salicylate, butyl salicylate, octyl salicylate (NEO HELIOPAN OS) or isooctyl salicylate and the like;

Examples of broad spectrum or UV A screening agents i.e. substances having absorption maxima between about 320 and 400 nm, which come into consideration for combination with the compounds of the present invention are for example the following organic and inorganic compounds :
--- Phenylene-1,4-bis-benzimidazolsulfonic acids or salts such as 2,2-(1,4-phenylene)bis-(1H-benzimidazol-4,6-disulfonic acid) (Neoheliopan AP)
--- Pigments such as microparticulated ZnO or TiO₂ and the like. The term "microparticulated" refers to a particle size from about 5 nm to about 200 nm, particularly from about 15 nm to about 100 nm. The particles may also be coated by other metal oxides such as e.g. aluminum or zirconium oxides or by organic coatings such as e.g. polyols, methicone, aluminum stearate, alkyl silane. Such coatings are well known in the art.

Component c) of the compositions of the present invention is a carrier for the UV filters a), b) and d). Suitable carriers for cosmetic bases are known to the skilled person, and usually the base will be a two phase system comprising a fatty phase and an aqueous phase. The base is suitably chosen, so that the final composition is e.g. a liquid or a solid oil-in-water emulsion, water-in-oil emulsion, multiple emulsion, microemulsion, PIT emulsion, pickering emulsion, hydrogel, alcoholic gel, lipogel, one or multiple phase solvents, ointments, suspensions, crèmes or other compositions. The base can also be selected to provide a water-free composition such as oil or a balsam, for example with vegetable or animal oils, mineral oils, synthetic oils or mixtures thereof. For these compositions, the corresponding oils or mixtures thereof are the carriers of component c) of the compositions of the present application. Other suitable carriers are for example fats, oils, waxes, silicon, alcohols and water and the preferred cosmetic bases mentioned below. Solid compositions such as soaps and powders are also possible, and suitable bases are known to the skilled person.

The compositions of the invention can also contain usual cosmetic adjuvants and additives. Thus, the carrier compounds and the usual adjuvants and additives include preservatives/antioxidants, fatty substances/ oils, water, organic solvents, silicones, thickeners, softeners, emulsifiers, additional sunscreens, antifoaming agents, moisturizers, fragrances, surfactants, fillers, sequestering agents, anionic, cationic, nonionic or amphoteric polymers or mixtures thereof, propellants, acidifying or basifying agents, dyes, colorants, pigments or nanopigments, in particular those suited for providing a photoprotective effect by physically blocking out ultraviolet radiation, or any other ingredients usually formulated into cosmetics, in particular for the production of sunscreen/ antisun compositions. The necessary amounts of the cosmetic and dermatological adjuvants and additives can, based on the desired product, easily be chosen by a skilled artisan in this field and will be illustrated in the examples, without being limited hereto.

An additional amount of antioxidants/ preservatives is generally preferred. Based on the invention all known antioxidants usually formulated into cosmetics can be used. Especially preferred are antioxidants chosen from the group consisting of amino acids (e.g. glycine, histidine, tyrosine, tryptophane) and their derivatives, imidazole (e.g. urocanic acid) and derivatives, peptides such as D,L-carnosine, D-carnosine, L-carnosine and derivatives (e.g. anserine), carotenoids, carotenes (e.g. α-carotene, β-carotene, lycopene) and derivatives, chlorogenic acid and derivatives, lipoic acid and derivatives (e.g. dihydrolipoic acid), aurothioglucose, propylthiouracil and other thiols (e.g. thioredoxine, glutathione, cysteine, cystine, cystamine and its glycosyl-, N-acetyl-, methyl-, ethyl-, propyl-, amyl-, butyl- and lauryl-, palmitoyl-; oleyl-, y-linoleyl-, cholesteryl- and glycerylester) and the salts, thereof, dilaurylthiodipropionate, distearylthiodipropionate, thiodipropionic acid and its derivatives (ester, ether, peptides, lipids, nucleotides, nucleosides and salts) as well as sulfoximine compounds (such as buthioninsulfoximine, homocysteinsulfoximine, buthioninsulfone, penta-, hexa-, heptathioninsulfoximine) in very low compatible doses (e.g. pmol bis µmol/kg), additionally (metal)-chelators (such as α-hydroxyfatty acids, palmic-, phytinic acid, lactoferrin), β-hydroxyacids (such as citric acid, lactic acid, malic acid), huminic acid, gallic acid, gallic extracts, bilirubin, biliverdin, EDTA, EGTA and its derivatives, unsaturated fatty acids and their derivatives (such as γ-linoleic acid, linolic acid, oleic acid), folic acid and its derivatives, ubiquinone and ubiquinol and their derivatives, vitamin C and derivatives (such as ascorbylpalmitate and ascorbyltetraisopalmitate, Mg-ascorbylphosphate, Na- ascorbylphosphate, ascorbylacetate), tocopherole and derivates (such as vitamin-E-acetate), mixtures of nat. vitamin E, vitamin A and derivatives (vitamin-A-palmitate and -acetate) as well as coniferylbenzoat, rutinic acid and derivatives, α-glycosylrutin, ferulic acid, furfurylidenglucitol, carnosin, butylhydroxytoluene, butylhydroxyanisole, trihydroxybutyrophenone, urea and its derivatives, mannose and derivatives, zinc and derivatives (e.g. ZnO, ZnSO₄), Selen and derivatives (e.g. selenomethionin), stilbenes and derivatives (such as stilbenoxide, trans-stilbenoxide) and suitable derivatives (salts, esters, ethers, sugars, nucleotides, nucleosides, peptides and lipids) of the named active ingredients. One or more preservatives/antioxidants may be present in an amount about 0.01 wt.% to about 10 wt.% of the total weight of the composition of the present invention. Preferably, one or more preservatives/antioxidants are present in an amount about 0.1 wt.% to about 1 wt.%.

Typically formulations also contain surface active ingredients like emulsifiers, solubilizers and the like. An emulsifier enables two or more immiscible components to be combined homogeneously. Moreover, the emulsifier acts to stabilize the composition. Emulsifiers that may be used in the present invention in order to form O/W, W/O, O/W/O or W/O/W emulsions/ microemulsions include those mentioned in the table(s) of examples as well as sorbitan oleate, sorbitan sesquioleate, sorbitan isostearate, sorbitan trioleate, polyglyceryl-3-diisostearate, polyglycerol esters of oleic/isostearic acid, polyglyceryl-6 hexaricinolate, polyglyceryl-4-oleate, polygylceryl-4 oleate/PEG-8 propylene glycol cocoate, oleamide DEA, TEA myristate, TEA stearate, magnesium stearate, sodium stearate, potassium laurate, potassium ricinoleate, sodium cocoate, sodium tallowate, potassium castorate, sodium oleate, and mixtures thereof. Further suitable emulsifiers are phosphate esters and the salts thereof such as cetyl phosphate (Amphisol^{®} A), diethanolamine cetyl phosphate (Amphisol^{®}), potassium cetyl phosphate (Amphisol^{®} K), sodium glyceryl oleate phosphate, hydrogenated vegetable glycerides phosphate and mixtures thereof. Furthermore, one or more synthetic polymers may be used as an emulsifier. For example, PVP eicosene copolymer, acrylates/C₁₀₋₃₀ alkyl acrylate crosspolymer, acrylates/steareth-20 methacrylate copolymer, PEG-22/dodecyl glycol copolymer, PEG-45/dodecyl glycol copolymer, and mixtures thereof. The preferred emulsifiers are cetyl phosphate (Amphisol^{®} A), diethanolamine cetyl phosphate (Amphisol^{®}), potassium cetyl phosphate (Amphisol^{®} K), PVP Eicosene copolymer, acrylates/C₁₀₋₃₀-alkyl acrylate crosspolymer, PEG-20 sorbitan isostearate, sorbitan isostearate, and mixtures thereof. The one or more emulsifiers are present in a total amount about 0.01 wt.% to about 20 wt.% of the total weight of the composition of the present invention. Preferably, about 0.1 wt.% to about 10 wt.% of emulsifiers are used.

The lipid phase of the composition of the invention can advantageously be chosen from:
mineral oils and mineral waxes;
oils such as triglycerides of caprinic acid or caprylic acid, preferable castor oil;
oils or waxes and other natural or synthetic oils, in an preferred embodiment esters of fatty acids with alcohols e.g. isopropanol, propyleneglycol, glycerine or esters of fatty alcohols with carbonic acids or fatty acids;
alkylbenzoates; and/or
silicone oils such as dimethylpolysiloxane, diethylpolysiloxane, diphenylpolysiloxane, cyclomethicones and mixtures thereof.

Exemplary fatty substances which can be incorporated in the oil phase of the emulsion, microemulsion, oleo gel, hydrodispersion or lipodispersion of the present invention are advantageously chosen from esters of saturated and/or unsaturated, linear or branched alkyl carboxylic acids with 3 to 30 carbon atoms, and saturated and/or unsaturated, linear and/or branched alcohols with 3 to 30 carbon atoms as well as esters of aromatic carboxylic acids and of saturated and/or unsaturated, linear or branched alcohols of 3-30 carbon atoms. Such esters can advantageously be selected from octylpalmitate, octylcocoate, octylisostearate, octyldodecylmyristate, cetearylisononanoate, isopropylmyristate, isopropylpalmitate, isopropylstearate, isopropyloleate, n-butylstearate, n-hexyllaureate, n-decyloleat, isooctylstearate, isononylstearate, isononylisononanoate, 2-ethyl hexylpalmitate, 2-ethylhexyllaurate, 2-hexyldecylstearate, 2-octyldodecylpalmitate, stearylheptanoate, oleyloleate, oleylerucate, erucyloleate, erucylerucate, tridecylstearate, tridecyltrimellitate, as well as synthetic, half-synthetic or natural mixtures of such esters e.g. jojoba oil.

Other fatty components suitable for use in the formulation of the present invention include polar oils such as lecithines and fatty acid triglycerides, namely triglycerol esters of saturated and/or unsaturated, straight or branched carboxylic acid with 8 to 24 carbon atoms, preferably of 12 to 18 carbon-atoms whereas the fatty acid triglycerides are preferably chosen from synthetic, half synthetic or natural oils (e.g. cocoglyceride, olive oil, sun flower oil, soybean oil, peanut oil, rape seed oil, sweet almond oil, palm oil, coconut oil, castor oil, hydrogenated castor oil, wheat oil, grape seed oil, macadamia nut oil and others); apolar oils such as linear and/ or branched hydrocarbons and waxes e.g. mineral oils, vaseline (petrolatum); paraffins, squalan and squalen, polyolefines, hydrogenated polyisobutenes and isohexadecanes, favored polyolefines are polydecenes; dialkyl ethers such as dicaprylylether; linear or cyclic silicone oils such as preferably cyclomethicone (octamethylcyclotetrasiloxane; cetyldimethicone, hexamethylcyclotrisiloxane, polydimethylsiloxane, poly(methy4phenylsiloxane) and mixtures thereof.

Other fatty components which can advantageously be incorporated in formulations of the present invention are isoeikosane; neopentylglykoldiheptanoate; propylenglykoldicaprylate/ dicaprate; caprylic/ capric/ diglycerylsuccinate; butylenglykol caprylat/caprat; C₁₂₋₁₃-alkyllactate; di-C₁₂₋₁₃ alkyltartrate; triisostearin; dipentaerythrityl hexacaprylat/hexacaprate; propylenglykolmonoisostearate; tricaprylin; dimethylisosorbid. Especially beneficial is the use of mixtures C₁₂₋₁₅-alkylbenzoate and 2-ethylhexylisostearate, mixtures C₁₂₋₁₅-alkylbenzoate and isotridecylisononanoate as well as mixtures of C₁₂₋₁₅-alkylbenzoate, 2-ethylhexylisostearate and isotridecylisononanoate.

The oily phase of the formulation of the present invention can also contain natural vegetable or animal waxes such as bee wax, china wax, bumblebee wax and other waxes of insects as well as shea butter and cocoa butter.

A moisturizing agent may be incorporated into a composition of the present invention to maintain hydration or rehydrate the skin. Moisturizers that prevent water from evaporating from the skin by providing a protective coating are called emollients. Additionally an emollient provides a softening or soothing effect on the skin surface and is generally considered safe for topical use. Preferred emollients include mineral oils, lanolin, petrolatum, capric/caprylic triglyceraldehydes, cholesterol, silicones such as dimeticone, cyclometicone, almond oil, jojoba oil, avocado oil, castor oil, sesame oil, sunflower oil, coconut oil and grape seed oil, cocoa butter, olive oil, aloe extracts, fatty acids such as oleic and stearic, fatty alcohols such as cetyl and hexadecyl (ENJAY), diisopropyl adipate, hydroxybenzoate esters, benzoic acid esters of C₉₋₁₅-alcohols, isononyl iso-nonanoate, ethers such as polyoxypropylene butyl ethers and polyoxypropylene cetyl ethers, and C₁₂₋₁₅-alkyl benzoates, and mixtures thereof. The most preferred emollients are hydroxybenzoate esters, aloe vera, C₁₂₋₁₅-alkyl benzoates, and mixtures thereof. An emollient is present in an amount of about 1 wt.% to about 20 wt.% of the total weight of the composition. The preferred amount of emollient is about 2 wt.% to about 15 wt.%, and most preferably about 4 wt.% to about 10 wt.%.

Moisturizers that bind water, thereby retaining it on the skin surface are called humectants. Suitable humectants can be incorporated into a composition of the present invention such as glycerin, polypropylene glycol, polyethylene glycol, lactic acid, pyrrolidon carboxylic acid, urea, phopholipids, collagen, elastin, ceramides, lecithin sorbitol, PEG-4, and mixtures thereof. Additional suitable moisturizers are polymeric moisturizers of the family of water soluble and/ or swellable/ and/ or with water gelating polysaccharides such as hyaluronic acid, chitosan and/or a fucose rich polysaccharide which is e.g. available as Fucogel®1000 (CAS-Nr. 178463-23-5) by SOLABIA S. One or more humectants are optionally present at about 0.5 wt.% to about 8 wt.% in a composition of the present invention, preferably about 1 wt.% to about 5 wt.%.

The aqueous phase of the preferred compositions of the present invention can contain the usual cosmetic additives such as alcohols, especially lower alcohols, preferably ethanol and/ or isopropanol, low diols or polyols and their ethers, preferably propyleneglycol, glycerine, ethyleneglycol, ethyleneglycol monoethyl- or monobutytether, propyleneglycol monomethyl- or -monoethyl- or-monobutylether, diethyleneglycol monomethyl-or monoethylether and analogue products, polymers, foam stabilisators; electrolytes and especially one or more thickeners. Thickeners that may be used in formulations of the present invention to assist in making the consistency of a product suitable include carbomer, siliciumdioxide, magnesium and/ or aluminum silicates, beeswax, stearic acid, stearyl alcohol polysaccharides and their derivatives such as xanthan gum, hydroxypropyl cellulose, polyacrylamides, acrylate crosspolymers preferably a carbomer, such as carbopole^{®} of type 980, 981, 1382, 2984, 5984 alone or mixtures thereof. Suitable neutralizing agents which may be included in the composition of the present invention to neutralize components such as e.g. an emulsifier or a foam builder/stabilizer include but are not limited to alkali hydroxides such as a sodium and potassium hydroxide; organic bases such as diethanolamine (DEA), triethanolamine (TEA), aminomethyl propanol, and mixtures thereof; amino acids such as arginine and lysine and any combination of any foregoing. The neutralizing agent can be present in an amount of about 0.01 wt.% to about 8 wt.% in the composition of the present invention, preferably, 1 wt.% to about 5 wt.%.

The addition of electrolytes into the composition of the present invention may be necessary to change the behavior of a hydrophobic emulsifier. Thus, the emulsions/microemulsions of this invention may contain preferably electrolytes of one or several salts including anions such as chloride, sulfates, carbonate, borate and aluminate, without being limited thereto. Other suitable electrolytes can be on the basis of organic anions such as, but not limited to, lactate, acetate, benzoate, propionate, tartrate and citrate. As cations preferably ammonium, alkylammonium, alkali- or alkaline earth metals, magnesium-, iron- or zinc-ions are selected. Especially preferred salts are potassium and sodium chloride, magnesium sulfate, zinc sulfate and mixtures thereof. Electrolytes can be present in an amount of about 0.01 wt.% to about 8 wt.% in the composition of the present invention.

The cosmetic compositions of the invention are useful as compositions for photoprotecting, the human epidermis or hair against the damaging effect of ultraviolet irradiation, as sunscreen compositions. Such compositions can, in particular, be provided in the form of a lotion, a thickened lotion, a gel, a scream, a milk, an ointment, a powder, a spray, a foam or a solid tube stick and can be optionally be packaged as an aerosol and can be provided in the form of a mousse, foam or a spray. When the cosmetic composition according to the invention are provided for protecting the human epidermis against UV radiation or as sunscreen composition, they can be in the form of a suspension or dispersion in solvents or fatty substances, or alternatively in the form of an emulsion or microemulsion (in particular of O/W or W/O type, O/W/O or W/O/W-type), such as a cream or a milk, a vesicular dispersion, in the form of an ointment, a gel, a solid tube stick or an aerosol mousse. The emulsions can also contain anionic, nonionic, cationic or amphoteric surfactants.

The following examples are provided to further illustrate the processes and compositions of the present invention. These examples are illustrative only and are not intended to limit the scope of the invention in any way.

**Example 1:**

| | 1 | Ref. | 2 | Ref. | 3 | Ref. | 4a | 4b | 4c | Ref. | 5 | Ref. | 6 | Ref. |
|---|---|---|---|---|---|---|---|---|---|---|---|---|---|---|
| | O/W milk | | O/W milk | | O/W milk | | O/W cream | | | | spray | | foam | |
| POLYGLYCERYL-2 DIPOLYHYDROXYSTEARATE | | | | | | | | | | | | | 2,0 | 4,0 |
| SORBITAN STEARATE | | | | | | | 2,0 | 2,0 | 2,0 | 2,0 | | | | |
| POLYGLYCERYL-2 SESQUIISOSTEARATE | <1,0 | | | <1,0 | | | | | | | | | | |
| GLYCERYL STEARATE SE | | | | | | | | | | | | | | |
| POLYSORBATE 60 | | | | | | | 1,5 | 1,5 | 1,5 | 1,5 | | | | |
| POLYGLYCERYL-3 POLYRICINOLEATE | | | | | | | | | | | | | | |
| LAURETH-4 PHOSPHATE | <1,0 | | | <1,0 | | | | | | | | | | |
| TRILAURETH-4 PHOSPHATE | | 1,5 | 1,5 | | 1,5 | 1,5 | | | | | | | | |
| SODIUM CETEARYL SULFATE | | | | | | | | | | | | | | |
| CETEARETH-20 | | | | | | | | | | | 1,0 | 1,0 | | |
| PEG-40 CASTOR OIL | | | | | | | | | | | | | | |
| COCOGLYCERIDES | | | | | | | | | | | | | | 5,0 |
| CAPRYLIC/CAPRIC TRIGLYCERIDE | | | | | | | | | | | | | 13,0 | |
| ETHYLHEXYL STEARATE | 4,0 | 5.0 | 4,0 | | 4,0 | 4,0 | | | | | | | | |
| DICAPRYLYL CARBONATE | | | | | | | 10,0 | 10,0 | 10,0 | 6,0 | 8,0 | 5,5 | | 2,0 |
| CERA MICROCRISTALLINA | | | | | | | 2,5 | 2,5 | 2,5 | 2,5 | | | | |
| MACADAMIA TERNIFOLIA | | | | | | | 1,0 | 1,0 | 1,0 | 1,0 | | | | |
| PRUNUS DULCIS | | | | | | | | | | | | | | |
| CETYL DIMETHICONE | | <1,0 | | | | | | | | | | | | |
| DIMETHICONE | | | | | | | <1,0 | <1,0 | <1,0 | <1,0 | | | | |
| CYCLOMETHICONE | | | | | | | 2,0 | 2,0 | 2,0 | 2,0 | 4,0 | 4,0 | | |
| PARAFFINUM LIQUIDUM | 4,0 | 5,0 | 4,0 | 4,0 | 4,0 | 4,0 | | | | | | | | |
| C12-15 ALKYL BENZOATE | 4,0 | | 4,0 | 4,0 | 4,0 | 4,0 | | | | | 7,5 | 5,0 | | |
| ISOPROPYL PALMITATE | | | | | | | | | | | | | | |
| STEARYL ALCOHOL | | | | | | | | | | | | | | |
| CETEARYL ALCOHOL | 2,0 | 2,0 | 2,0 | 2,0 | 2,0 | 2,0 | 3,0 | 3,0 | 3,0 | 2,0 | | | | |
| Polysilicone-15 (Parsol SLX) | 2,5 | | 1.0 | | 2,5 | | 2,5 | 2,5 | 2,5 | | 2,5 | 2,5 | 2,5 | |
| BUTYL METHOXYDIBENZOYLMETHANE | 1,5 | 1,5 | 1,5 | 1,3 | 2,0 | 2,0 | 3,0 | 3,0 | | 3,0 | 3,0 | 3,0 | 3,0 | 4,0 |
| 4-METHYLBENZYLIDENE CAMPHOR | 4,0 | | 3,5 | | 4,0 | 4,0 | 4,0 | | | 4,0 | 4,0 | 4,0 | 4,0 | |
| OCTOCRYLENE | | 1,0 | | | | | | | | | | | | 3,2 |
| ETHYLHEXYL METHOXYCINNAMATE | | | | 8,0 | 5,0 | 5,0 | | | | 7,0 | | 5,0 | | 10,0 |
| TITANIUM DIOXIDE | | 1,5 | | | | | | | | 1,5 | | | | |
| PHENYLBENZIMIDAZOLE SULFONIC ACID | 1,5 | 2,0 | 0,5 | 1,40 | 2,0 | 2,0 | 2,0 | 2,0 | 2,0 | 2,5 | 2,0 | 2,0 | 2,5 | 2,5 |
| 2-(4-Diethylamino-2-hydroxybenzoyl)-benzoic acid hexylester | | | | | | | | | 3,0 | | | | | |
| PANTHENOL | | | | | | | 1,0 | 1,0 | 1,0 | 1,0 | | | 1,0 | 1,0 |
| GLYCERIN | 5,0 | 5,0 | 5,0 | 5,0 | 5,0 | 5,0 | 5,0 | 5,0 | 5,0 | 5,0 | 5,0 | 5,0 | 5,0 | 5,0 |
| GALACTOARABINAN | | | | | | | | | | | <1,0 | <1,0 | | |
| XANTHAN GUM | <1,0 | <1.0 | <1,0 | <1.0 | <1,0 | <1,0 | <1,0 | <1,0 | <1,0 | <1,0 | | | | |
| CARBOMER | <1,0 | <1,0 | <1,0 | <1,0 | <1,0 | <1,0 | 1,0 | 1,0 | 1,0 | 1,0 | <1,0 | <1,0 | | |
| ACRYLATES/C10-30ALKYL ACRYLATE CROSSPOLYMER | | <1,0 | | | | | <1,0 | <1,0 | <1,0 | <1,0 | <1,0 | <1,0 | | |
| DISODIUM EDTA | <1,0 | <1,0 | <1,0 | <1,0 | <1,0 | <1,0 | <1,0 | <1,0 | <1,0 | <1,0 | <1,0 | <1,0 | <1,0 | <1,0 |
| DISODIUM PEG-5 LAURYLCITRATE SULFOSUCCINATE, SODIUM LAURETH SULFATE | | | | | | | | | | | | | <1,0 | <1,0 |
| CAPRYL/CAPRAMIDOPROPYL BETAINE | | | | | | | | | | | | | <1,0 | <1,0 |
| PHYTANTRIOL | <1,0 | <1,0 | <1,0 | <1,0 | <1,0 | <1,0 | <1,0 | <1,0 | <1,0 | <1,0 | <1,0 | <1,0 | <1,0 | <1,0 |
| TOCOPHERYLACETATE | <1,0 | <1,0 | <1,0 | <1,0 | <1,0 | <1,0 | <1,0 | <1,0 | <1,0 | <1,0 | <1,0 | <1,0 | <1,0 | <1,0 |
| SODIUM ASCORBYL PHOSPHATE | <1,0 | <1,0 | <1,0 | <1,0 | <1,0 | <1,0 | <1,0 | <1,0 | <1,0 | <1,0 | <1,0 | <1,0 | <1,0 ' | <1,0 |
| ALCOHOL | 8,0 | 8,0 | 8,0 | 8,0 | 8,0 | 8,0 | 7,0 | 7,0 | 7,0 | 7,0 | | | 7,0 | 7,0 |
| Preservative | | | | | | | | | | | qs. | | | |
| Parfum, NaCl, NaOH | qs. | | | | | | | | | | | | | |
| AQUA | ad 100 | | | | | | | | | | | | | |
| Total UV filter content | 9,5 | 14,0 | 6,5 | 10,3 | 15,5 | 13,0 | 11,5 | 7,5 | 7,5 | 18,0 | 11,5 | 16,5 | 12,0 | 19,7 |
| SPF | 21 | 20 | 14 | 8 | 24 | 31 | 31 | 17 | 19 | 25 | 29 | 22 | 22 | 21 |
| Ratio SPF/∑ (UV-filter) | 2.21 | 1.43 | 2.15 | 0.78 | 1.55 | 2.38 | 2.70 | 2.27 | 2.53 | 1.39 | 2.52 | 1.33 | 1.83 | 1.07 |
| 1 to 6 = formula with polysifoxane-based UV filter | | | | | | | | | | | | | | |
| Ref. = comparative formula | | | | | | | | | | | | | | |

### Example 2:

The determination of the sun protecting factor was performed according to the COLIPA protocol (the European Cosmetic, Toiletry and Perfumery Association, Sun Protection Factor test method 1994). An application dose of 2 mg/ cm2 was used according to the COLIPA protocol for in vivo SPF measurements in humans, on an application area of 50 cm2. The following filter combinations have been formulated using suitable model O/W lotions as described below to yield stable sunscreen lotions.

| | Reference | | | 1 | 2 | 3 |
|---|---|---|---|---|---|---|
| Polysilicone 15 (Parsol SLX) | 4 | 5 | 5 | 4 | 4 | 2.5 |
| Butyl methoxydibenzoylmethane | | | | 2 | | 2.5 |
| Tinosorb M (50% active) | | | | | 4 (2) | |
| Phenylbenzimidazolsulfonic acid | | | 1.1 | | | |
| 4-Methylbenzylidene champhor | | | | | | 4 |
| Ethylhexy methoxycinnamate | | 1 | | | | |
| Silicone 2503 Cosmetic Wax | | | | | 2 | 2 |
| Dimethicone 200/100 | | 4 | 4 | | | |
| Tegosoft TN | 15 | | | | | |
| Miglyol 812 | | | | 15 | 15 | 15 |
| Cetyl Alcohol | 2 | | | 1 | 1 | 1 |
| PPG-3 Myristylether | | 17.5 | 17.5 | | | |
| Sorbitan Stearate | | 2.5 | 2.5 | | | |
| Stearyl alcohol | | 1 | 1 | | | |
| BHT | 0.05 | | | 0.05 | 0.05 | 0.05 |
| Estol GMM 3650 | 4 | | | 3 | 3 | 3 |
| Polysorbate 60 | | 2.5 | 2.5 | | | |
| Edeta BD | 0.1 | | | 0.1 | 0.1 | 0.1 |
| Phenonip | 0.6 | | | 0.6 | 0.6 | 0.6. |
| Amphisol A | 2 | | | 2 | 2 | 2 |
| Tris amino 25% sol. | | | | 2 | 2 | 2 |
| Xanthan Gum | | 0.3 | 0.3 | | | |
| Carbopol EDT 2001 | 0.3 | | | 0.2 | 0.2 | 0.2 |
| Polyacrylamid &C13-14 isoparraffn+laureth 7 | | 2 | 2 | | | |
| Propylene glycol | 5 | | | | | |
| Glycerin | | | | 3 | 3 | 3 |
| Tris amino 25% sol. | 4.5 | | | 2 | 2 | 2 |
| Vitamine E Acteate | 2 | | | | | |
| Perfume, NaCl, NaOH | q.s. | | | | | |
| aqua | ad 100 | | | | | |
| Total UV filter content | 4 | 6 | 6.1 | 6 | 6 | 9 |
| SPF | 3 | 5.8 | 8.2 | 13 | 13 | 17 |
| Ratio SPF/ Σ (UV-filter) | 0.8 | 1.0 | 1.3 | 2.2 | 2.2 | 1.9. |

The combination of the polysiloxane and one or several UV filter containing appropriate bulky (sterically demanding) substituents according to the invention show an unproportional increase of the SPF and thus a significantly increased ratio of the *sunprotecting factor* to the *total amount of UV filters* of the light protecting composition. This effect can also be observed if the light protecting composition contains optionally additional UV-filter.

## Claims

1. Light protecting composition comprising
a) at least one polysiloxane-based UV filter,
b) at least one additional UV filter which chromophore contains appropriate bulky (sterically demanding) substituents,
wherein the UV filter(s) containing bulky substituents is (are) selected from the group consisting of 2-(4-diethylamino-2-hydroxy-benzoyl)-benzoic acid hexylester, 4-methyl benzylidenecamphor, 3-benzylidenecamphor, homosalate, benzylidenecamphor sulfonic acid, methylene bis-benzotriazo tetramethylbutylphenol, drometrizole trisiloxane, and camphor benzalkonium methosulfate,
c) a carrier for the components a) and b)
with the proviso that 4,4'4"-(1,3,5-triazine-2,4,6-triyltriimino)-tris-benzoic-acid-tris(2-ethylhexylester) is not present in the composition.

2. Light protecting composition according to claim 1, wherein the polysiloxane-based UV filter is a compound according to formula Ia or Ib: 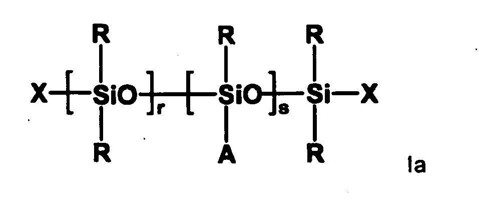 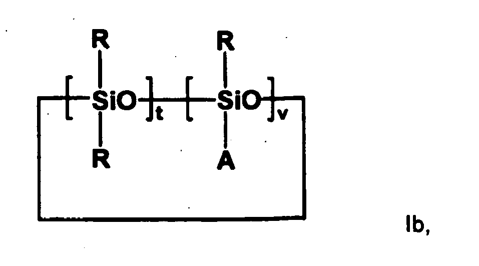 wherein
X is R or A;
A is selected from formula IIa, IIb or IIc: 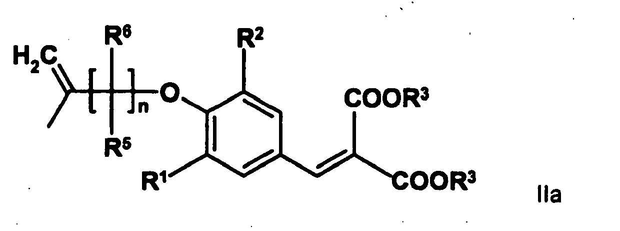 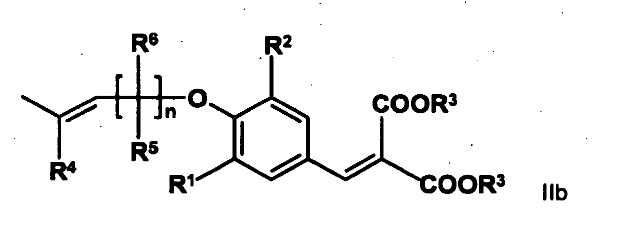 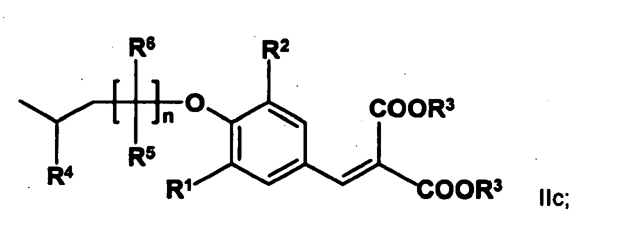
R is hydrogen, C₁₋₆alkyl or phenyl;
R¹ and R² are each independently hydrogen, hydroxy, C₁₋₆-alkyl or C₁₋₆-alkoxy;
R³ is C₁₋₆-alkyl;
R⁴ is hydrogen or C₁₋₆-alkyl;
R⁵ and R⁶ are each independently hydrogen or C₁₋₆-alkyl;
r is from 0 to 250;
s is from 0 to 20;
r + s is at least 3;
t is from 0 to 10;
v is from 0 to 10;
v + t is at least 3; and
n is from 1 to 6;
with the proviso that when s is 0, at least one X is A.

3. Light protecting composition according to claim 2, wherein
X is methyl,
A is a group of the formula IIa or IIb,
R is methyl,
R¹ and R² are each hydrogen,
R³ is ethyl,
R⁴ is hydrogen,
R⁵ and R⁶ are hydrogen,
r is a statistical mean value of about 60,
s is a statistical mean value of about 4 and
n is 1.

4. Light protecting composition according to claim 1 wherein the sum-amount of all UV filters a) is lower or equal to the sum-amount of all UV filters b).

5. Method to increase the ratio of the *sunprotecting factor* to the *total UV filter amount,* the method comprising
a) the addition of a polysiloxane-based UV filter in order to reduce the amount of a UV filter which is liquid at room temperature (25°C) by which the total UV filter amount will be reduced, and
b) the addition of UV filter(s) containing bulky groups wherein the UV filter(s) containing bulky substituents is (are) selected from the group consisting of 2-(4-Diethylamino-2-hydroxy-benzoyl)-benzoic acid hexylester, 4-methyl benzylidene camphor, 3-benzylidenecamphor, homosalate, benzylidenecamphor sulfonic acid, methylene bis-benzotriazo tetramethylbutylphenol, drometrizole trisiloxane and camphor benzalkonium methosulfate, and optionally
c) the addition of UV filter(s) which are not liquid at room temperature (25°C) in order to increase the sunprotecting factor of the light protecting composition.

6. Method according to claim 5, wherein the UV filter which is liquid at room temperature (25°C) is selected from the group consisting of octocrylene, ethylhexyl methoxycinnamate, PEG-25 PABA, isoamyl p-methoxycinnamate and octyl dimethyl PABA.

7. Method according to any of claims 5 or 6, wherein the UV filter(s) which is not liquid at room temperature (25°C) is selected from the group consisting of, phenylbenzimidazole sulfonic acid, disodium phenyl dibenzimidazole, tetrasulfonate ethylhexy triazone, diethylhexyl butamido triazone, bis-ethylhexyloxyphenol methoxyphenyl triazine, benzophenone- 3 and/ or benzophenone-4 , TiO₂ and ZnO.

## Patentansprüche

1. Lichtschutzzusammensetzung, umfassend
a) mindestens einen Polysiloxan-basierenden UV-Filter,
b) mindestens einen zusätzlichen UV-Filter, dessen Chromophor geeignete große (sterisch anspruchsvolle) Substituenten enthält,
wobei der/die UV-Filter, der/die große(n) Substituenten enthält/enthalten, ausgewählt ist/sind aus der Gruppe, bestehend aus 2-(4-Diethylamino-2-hydroxy-benzoyl)-benzoesäure-hexylester, 4-Methyl-benzyliden-kampfer, 3-Benzyliden-kampfer, 3,3,5-Trimethylcyclohexylsalicylat, Benzylidenkampfersulfonsäure, Methylen-bis-benzotriazo-tetramethyl-butylphenol, Drometrizol-trisiloxan und Kampfer-benzalkonium-methosulfat,
c) einen Träger für die Komponenten a) und b),
mit der Maßgabe, dass 4,4',4"-(1,3,5-Triazin-2,4,6-triyltriimino)-tris-benzoesäure-tris-(2-ethylhexylester) in der Zusammensetzung nicht vorliegt.

2. Lichtschutzzusammensetzung nach Anspruch 1, wobei der Polysiloxan-basierende UV-Filter eine Verbindung nach Formel Ia oder Ib ist: 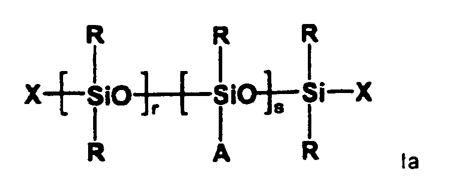 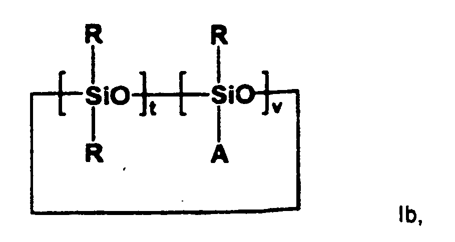 worin
X R oder A ist;
A aus den Formeln IIa, IIb oder IIc ausgewählt ist: 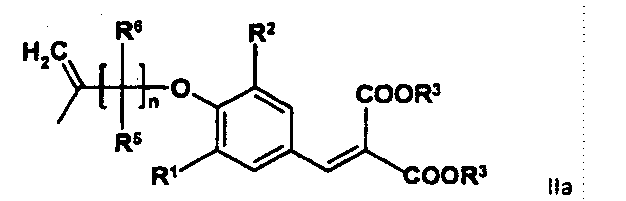 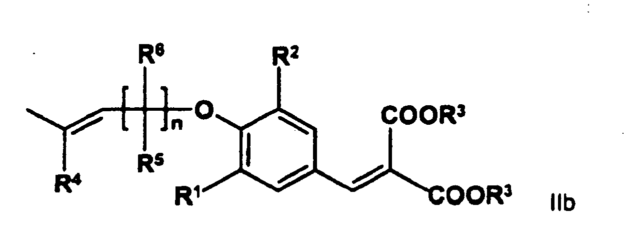 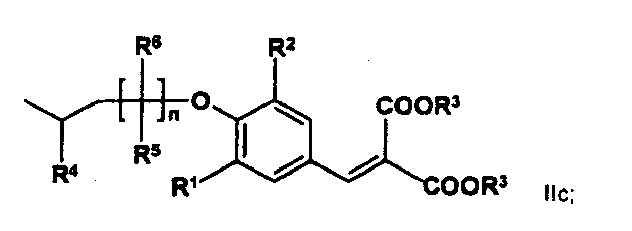
R Wasserstoff, C₁₋₆-Alkyl oder Phenyl ist;
R¹ und R² jeweils unabhängig voneinander Wasserstoff, Hydroxy, C₁₋₆-Alkyl oder C₁₋₆-Alkoxy sind;
R³ C₁₋₆-Alkyl ist;
R⁴ Wasserstoff oder C₁₋₆-Alkyl ist;
R⁵ und R⁶ jeweils unabhängig voneinander Wasserstoff oder C₁₋₆-Alkyl sind;
r 0 bis 250 ist;
s 0 bis 20 ist;
r + s mindestens 3 ist;
t 0 bis 10 ist;
v 0 bis 10 ist;
v + t mindestens 3 ist; und
n 1 bis 6 ist;
mit der Maßgabe, dass, wenn s 0 ist, mindestens ein X A ist.

3. Lichtschutzzusammensetzung nach Anspruch 2, worin
X Methyl ist,
A eine Gruppe der Formel IIa oder IIb ist,
R Methyl ist,
R¹ und R² jeweils Wasserstoff sind,
R³ Ethyl ist,
R⁴ Wasserstoff ist,
R⁵ und R⁶ Wasserstoff sind,
r ein statistischer Mittelwert von etwa 60 ist,
s ein statistischer Mittelwert von etwa 4 ist und
n 1 ist.

4. Lichtschutzzusammensetzung nach Anspruch 1, wobei die Summe aller UV-Filter a) kleiner oder gleich zu der Summe aller UV-Filter b) ist.

5. Verfahren zur Erhöhung des Verhältnisses des *Sonnenschutzfaktors* zur *UV-Filter-Gesamtmenge,* wobei das Verfahren
a) die Zugabe eines Polysiloxan-basierenden UV-Filters zur Verringerung der Menge eines UV-Filters, der bei Raumtemperatur (25 °C) flüssig ist, wodurch die UV-Filter-Gesamtmenge verringert wird, und
b) die Zugabe von UV-Filter(n), der/die große Gruppen enthält/enthalten, wobei der/die UV-Filter, der/die große Substituenten enthält, ausgewählt ist/sind aus der Gruppe, bestehend aus 2-(4-Diethylamino-2-hydroxy-benzoyl)-benzoesäure-hexylester, 4-Methyl-benzyliden-kampfer, 3-Benzyliden-kampfer, 3,3,5-Trimethylcyclohexylsalicylat, Benzylidenkampfersulfonsäure, Methylen-bis-benzotriazo-tetramethyl-butylphenol, Drometrizol-trisiloxan und Kampfer-benzalkonium-methosulfat, und gegebenenfalls
c) die Zugabe von UV-Filter(n), der/die bei Raumtemperatur (25 °C) nicht flüssig ist/sind, umfasst, um so den Sonnenschutzfaktor der Lichtschutzzusammensetzung zu erhöhen.

6. Verfahren nach Anspruch 5, wobei der UV-Filter, der bei Raumtemperatur (25 °C) flüssig ist, ausgewählt ist aus der Gruppe, bestehend aus Octocrylen, Ethylhexylmethoxycinnamat, PEG-25-PABA, Isoamyl-p-methoxycinnamat und Octyldimethyl-PABA.

7. Verfahren nach einem der Ansprüche 5 oder 6, wobei der/die UV-Filter, der/die bei Raumtemperatur (25 °C) nicht flüssig ist/sind, ausgewählt ist/sind aus der Gruppe, bestehend aus Phenylbenzimidazolsulfonsäure, Dinatriumphenyldibenzimidazol, Tetrasulfonatethylhexyltriazon, Diethylhexylbutamidotriazon, Bis-ethylhexyloxyphenolmethoxyphenyltriazin, Benzophenon-3 und/oder Benzophenon-4, TiO₂ und ZnO.

## Revendications

1. Composition de protection contre la lumière comprenant :
a) au moins un filtre UV à base de polysiloxane,
b) au moins un filtre UV supplémentaire, dont le chromophore contient des substituants volumineux (stériquement exigeants) appropriés, dans lequel le(s) filtre(s) UV contenant des substituants volumineux est (sont) choisi(s) dans le groupe constitué d'hexylester d'acide 2-(4-diéthylamino-2-hydroxy-benzoyl)-benzoïque, de 4-méthylbenzylidène-camphre, de 3-benzylidène-camphre, d'homosalate, d'acide benzylidène-camphre sulfonique, de bis-benzotriazotétraméthylbutylphénol de méthylène, de trisiloxane de drométrizole, et de méthosulfate de benzalkonium de camphre,
c) un excipient pour les composants a) et b), à condition que le tris(2-éthylhexylester) d'acide 4,4',4"-(1,3,5-triazine-2,4,6-triyltriimino)-tris-benzoïque ne soit pas présent dans la composition.

2. Composition de protection contre la lumière selon la revendication 1, dans laquelle le filtre UV à base de polysiloxane est un composé selon la formule Ia ou Ib : 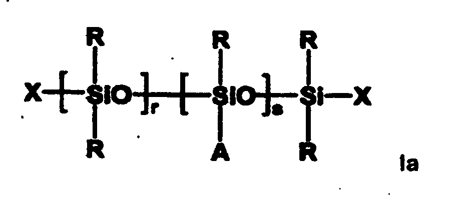 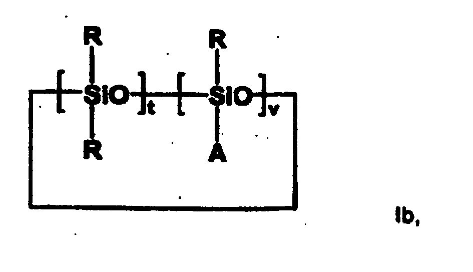 où
X est un R ou A ;
A est choisi parmi la formule IIa, IIb ou IIc : 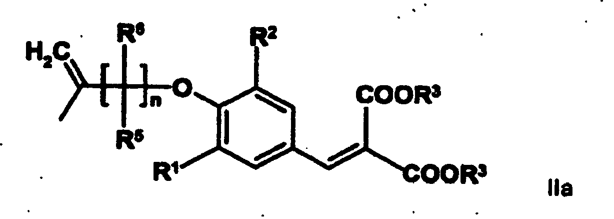 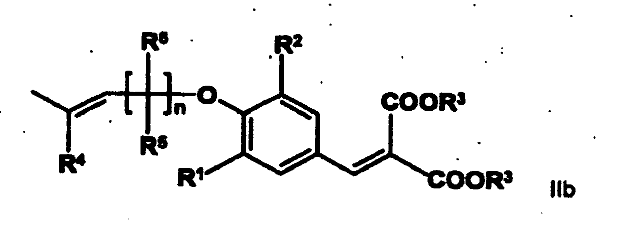 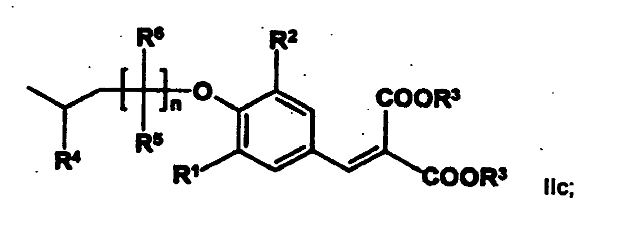 R est un hydrogène, un alkyle en C₁₋₆ ou un phényle ;
R¹ et R² sont chacun indépendamment un hydrogène, un hydroxy, un alkyle en C₁₋₆ ou un alcoxy en C₁₋₆; R³ est un alkyle en C₁₋₆ ;
R⁴ est un hydrogène ou un alkyle en C₁₋₆ ;
R⁵ et R⁶ sont chacun indépendamment un hydrogène ou un alkyle en C₁₋₆ ;
r est compris entre 0 et 250 ;
s est compris entre 0 et 20 ;
r + s est au moins égal à 3 ;
t est compris entre 0 et 10 ;
v est compris entre 0 et 10 ;
t + v est au moins égal à 3 ; et
n est compris entre 1 et 6 ;
à condition que, quand s est égal à 0, au moins un X soit A.

3. Composition de protection contre la lumière selon la revendication 2, dans laquelle
X est un méthyle
A est un groupe de formule IIa ou IIb,
R est un méthyle
R¹ et R² sont chacun un hydrogène,
R³ est un éthyle
R⁴ est un hydrogène
R⁵ et R⁶ sont un hydrogène
r est une valeur moyenne statistique d'environ 60
s est une valeur moyenne statistique d'environ 4 et
n est égal à 1.

4. Composition de protection contre la lumière selon la revendication 1, dans laquelle la somme-quantité de tous les filtres UV a) est inférieure ou égale à la somme-quantité de tous les filtres UV b).

5. Procédé d'augmentation du *rapport du facteur de protection solaire* sur *la quantité de filtre UV* totale, le procédé comprenant :
a) l'addition d'un filtre UV à base de polysiloxane afin de réduire la quantité de filtre UV qui est liquide à la température ambiante (25°C), réduisant ainsi la quantité de filtre UV totale, et
b) l'addition de filtre(s) UV contenant des groupes volumineux, dans laquelle le(s) filtre(s) UV contenant des substituants encombrants est (sont) choisi(s) dans le groupe constitué d'hexylester d'acide 2-(4-diéthylamino-2-hydroxybenzoyl)-benzoïque, de 4-méthyl-benzylidène-camphre, de 3-benzylidène-camphre, d'homosalate, d'acide benzylidène-camphre sulfonique, de bis-benzotriazo-tétraméthylbutylphénol de méthylène, de trisiloxane de drométrizole, et de méthosulfate de benzalkonium de camphre, et éventuellement
c) l'addition de filtre (s) UV qui n'est pas (ne sont pas) liquide(s) à la température ambiante (25°C), afin d'augmenter le facteur de protection solaire de la composition de protection contre la lumière.

6. Procédé selon la revendication 5, dans lequel le filtre UV qui est liquide à la température ambiante (25°C) est choisi dans le groupe constitué d'octocrylène, d'éthylhexyl méthoxycinnamate, de PEG-25 PABA, d'isoamyl p-méthoxycinnamate et d'octyl diméthyl PABA.

7. Procédé selon l'une quelconque des revendications 5 ou 6, dans lequel le(s) filtre(s) UV qui n'est pas liquide à la température ambiante (25°C) est choisi dans le groupe constitué d'acide phénylbenzimidazole sulfonique, de disodium phényl dibenzimidazole, de tétrasulfonate éthylhexyltriazone, de diéthylhexy butamido triazone, de bis-éthylhexyloxyphénol méthoxyphényl triazine, de benzophénone-3 et/ou de benzophénone-4, de TiO₂ et de ZnO.
